# EUROPEAN PATENT SPECIFICATION

(11) **EP 4 316 429 B1**
(45) Date of publication and mention of the grant of the patent: **18.11.2026**
(21) Application number: 22778517.7
(22) Date of filing: 10.03.2022
(51) Int. Cl.: A61F 2/82, A61F 2/07, A61F 2/90, A61F 2/856, A61F 2/954, A61F 2/966

(54) **COVERED STENT AND MEDICAL DEVICE**
ABGEDECKTER STENT UND MEDIZINISCHE VORRICHTUNG
STENT RECOUVERT ET DISPOSITIF MÉDICAL

(30) Priority: 31.03.2021 CN 202110348981
(43) Date of publication of application: 07.02.2024
(73) Proprietor: Shanghai MicroPort Endovascular MedTech (Group) Co., Ltd., Shanghai 201318 (CN)
(72) Inventor: ZHU, Yongfeng, Shanghai 201318 (CN); ZHANG, Bowei, Shanghai 201318 (CN); WU, Weiyi, Shanghai 201318 (CN); ZHAO, Mingjie, Shanghai 201318 (CN); ZHANG, Junli, Shanghai 201318 (CN); ZHU, Qing, Shanghai 201318 (CN)
(74) Representative: Patentship Patentanwaltsgesellschaft mbH
(86) International application number: PCT/CN2022/080167
(87) International publication number: WO 2022/206333

(56) References cited:
- WO-A1-2018/156847
- CN-A- 109 009 562
- CN-A- 110 022 795
- CN-A- 110 507 448
- CN-A- 112 022 436
- CN-U- 212 415 986
- CN-U- 212 415 986
- CN-U- 212 522 082
- CN-U- 212 522 100
- CN-U- 214 967 137
- US-A1- 2002 040 236
- US-A1- 2013 116 773
- US-A1- 2014 148 895
- US-A1- 2014 257 453

## Description

### TECHNICAL FIELD

The present invention relates to the technical field of medical instrument and, more specifically, to a covered stent and a medical device.

### BACKGROUND

Cardiovascular disease poses a great threat to human health, and especially aortic diseases such as aortic aneurysm is one of the most dangerous of acute and severe cardiovascular diseases. At present, the treatment methods for aortic diseases include drug treatment, surgical treatment, interventional treatment, etc. The principle of interventional treatment lies in that: the compressible stent is delivered to the diseased part of the blood vessel and then released, and after the stent is expanded in the blood vessel, the diseased part is isolated from the vascular cavity, so that blood can pass through the stent, thereby protecting the diseased blood vessel and achieving the purpose of repairing the diseased blood vessel. Interventional treatment is widely used because of its advantages in incurring less trauma, being safe and effective.

For aortic aneurysms or aortic dissections that do not involve the aortic arch, straight-tube stents can be used for interventional treatment, while for diseases involving the aortic arch, the use of only straight-tube stents to isolate the diseased part will cause a problem that branch vessels on the aortic arch are blocked. Generally, along a direction from the ascending aorta to the descending aorta, there are three important branch vessels on the aortic arch, which are the innominate artery (IA), the left common carotid artery (LCCA) and the left subclavian artery (LSA). If the IA and the LCCA are blocked directly, the patient will be dead. Therefore, developing a stent that adapts to the anatomy of the aortic arch and avoids the closing of the branch vessels on the aortic arch is an urgent problem to be solved.

CN 212522082 U discloses a covered stent. CN 212415986 U discloses a stent device and a stent system. US 2014/0257453 A1 discloses an endoluminal prosthesis with a graft. US 2013/0116773 A1 discloses an endoluminal prosthesis. US 2002/0040236 A1 discloses a medical device such as a foldable stent or stent-graft and a method of using it.

### SUMMARY OF THE INVENTION

An object of the present invention is to provide a covered stent and a medical device. The covered stent is adapted to the anatomy of the aortic arch. When the covered stent is implanted into the aortic arch, the diseased part can be isolated while branch vessels can be prevented from being blocked.

In order to achieve the above object, the present invention provides a covered stent, comprising a main stent and a first branch stent, wherein a first interface, a second interface and a third interface are provided on a side surface of the main stent and spaced along an axial direction of the main stent, the first interface is configured to receive the first branch stent, the second interface is configured to receive a second branch stent, and/or the third interface is configured to receive a third branch stent; wherein the covered stent further comprises a restraining assembly arranged on the main stent, and wherein the restraining assembly is configured to apply to the main stent a tensile force toward an inner cavity of the main stent, so that at least a portion of the main stent that is provided with the second interface and the third interface is recessed toward the inner cavity of the main stent.

Optionally, the portion of the main stent that is provided with the second interface and the third interface has an inner diameter for a recessed state that is 20% to 80% of an inner diameter for a natural state.

A collapsed area is formed at the side surface of the main stent, and the second interface and the third interface are both arranged in the collapsed area.

The first interface is located in a portion of the main stent other than the collapsed area, and the second interface and the third interface are located on two opposite side surfaces of the collapsed area.

Optionally, the collapsed area of the main stent has an inner diameter for a natural state that is 50% to 90% of a maximum inner diameter of the main stent.

The restraining assembly is disposed at least partially at the collapsed area, and is configured to apply the tensile force to the collapsed area and to cause the collapsed area to be further recessed toward the inner cavity of the main stent.

Optionally, the main stent comprises a first ring and a first membrane at least partially covering the first ring, and wherein the first ring comprises at least one first sub-ring and at least two second sub-rings, the at least two second sub-rings arranged on both axial sides of all of the at least one first sub-ring, and the at least one first sub-ring and the at least two second sub-rings form the collapsed area;
wherein the restraining assembly comprises a plurality of coils and a restraining wire, and wherein at least some of the coils are disposed in the collapsed area and connected to at least one of the at least one first sub-ring, and at least two of the coils are arranged circumferentially on one of the at least one first sub-ring along a circumferential direction of the main stent; the restraining wire passes through all the coils; when the restraining wire is tense, the tensile force is applied from the restraining wire to the collapsed area through the coils.

Optionally, the covered stent further comprises a second branch stent and/or a third branch stent, and wherein the second branch stent is configured to be detachably arranged at the second interface, and the third branch stent is configured to be detachably arranged at the third interface.

Optionally, a predetermined distance is defined between the second interface and any one of the at least one first sub-ring and at least two second sub-rings; and/or a predetermined distance is defined between the third interface and any one of the at least one first sub-ring and at least two second sub-rings.

Optionally, the covered stent further comprises a first embedded support and/or a second embedded support, and wherein the first embedded support is arranged at the first interface, is located in the inner cavity of the main stent, and is in communication with the inner cavity of the main stent; the second embedded support is arranged at the third interface, is located in the inner cavity of the main stent, and is in communication with the inner cavity of the main stent.

In order to achieve the above object, the present invention also provide a medical device, comprising a delivery mechanism and the above covered stent, wherein the delivery mechanism is configured to deliver and release the covered stent to a predetermined position.

As compared with the prior art, the covered stent and the medical device according to the present invention have the following advantages:
The covered stent includes a main stent and a first branch stent, the main stent has a side surface provided thereon with a first interface, a second interface and a third interface spaced along an axial direction of the main stent, the first interface is configured to receive the first branch stent, the second interface is configured to receive a second branch stent, and/or the third interface is configured to receive a third branch stent; the covered stent further includes a restraining assembly arranged on the main stent, and the restraining assembly is configured to apply to the main stent a tensile force toward an inner cavity of the main stent, so that at least a portion of the main stent that is provided with the second interface and the third interface is recessed toward the inner cavity of the main stent. During operation, when the covered stent is implanted into the aorta and the corresponding branch stent needs to be implanted at the second interface and/or the third interface, the restraining assembly applies a tensile force to the main stent, so that the portion where the second interface and the third interface are located is recessed toward the inner cavity of the main stent, which makes it convenient to establish the passageway for the branch stents, facilitates the implantation and release of branch stents, and avoids branch vessel ischemia caused from a blocking of the branch vessel during the implantation of the branch stents.

The portion of the main stent that is provided with the second interface and the third interface has an inner diameter in a recessed state that is 20% to 80% of an inner diameter in a natural state, which makes it convenient to implant branch stents and avoids blocking the branch blood vessels. Moreover, it ensures that the blood flow passing through the inner cavity of the main stent is sufficient to avoid ischemia of the lower extremities.

The collapse area is formed on a side surface of the main stent, and the second interface and the third interface are both arranged in the collapse area. In this way, the main stent can be recessed sufficiently under a small tensile force provided from the restraining assembly, so as to prevent the second interface and/or the third interface from becoming closed, which further facilitates the establishment of the passageway for the branch stents and simplifies the implantation operation of the branch stent. The degree of concavity of the area on the opposite side of the collapsed area under the action of the tensile force can be reduced, and thus the possibility of the beak phenomenon occurring during the subsequent complete release of the main stent. If one of the second interface and the third interface is not provided with a branch stent, blood flow can still flow through the collapsed area into the branch vessel.

### BRIEF DESCRIPTION OF THE DRAWINGS

The present invention will be better understood with reference to the accompanying drawings provided without limiting the invention, in which:
Fig. 1 is a schematic diagram showing a partial structure of human aorta;
Fig. 2 is a schematic diagram showing the structure of a covered stent according to an embodiment of the present invention, where a branch stent is not provided on the first interface or the third interface;
Fig. 3 is a schematic diagram showing a partial structure of a covered stent according to an embodiment of the present invention, where the restraining assembly applies a tensile force to the collapsed area;
Fig. 4a is an A-A sectional view of the covered stent shown in Fig. 2, where the first branch stent is omitted;
Fig. 4b is a B-B sectional view of the covered stent shown in Fig. 2;
Fig. 5 is a schematic diagram showing a partial structure of the anterior segment of the main stent of the covered stent according to an embodiment of the present invention, where the subfigure a) shows that the length of the second sub-ring in the entire circumference is equal, and the subfigure b) shows that the length of the second sub-ring gradually decreases along the direction from the first side to the second side;
Fig. 6 is a schematic diagram showing the structure of the proximal end of the main stent of the covered stent according to an embodiment of the present invention, where the subfigure a) shows that some of the proximal apexes of the second sub-ring located at the proximal end are not covered by the first membrane, and the subfigure b) shows that all of the proximal apexes of the second sub-ring located at the proximal end are not covered by the first membrane;
Fig. 7 is a schematic diagram showing the structure of the proximal end of the main stent of the covered stent according to an embodiment of the present invention, where at least some areas of the second sub-ring located at the proximal end are non-fixedly connected to the first membrane;
Fig. 8 is a schematic diagram showing the structure of the proximal end of the main stent of the covered stent according to an embodiment of the present invention, where the subfigure a) shows that some of the distal apexes of the second sub-ring located at the distal end are not covered by the first membrane, and the subfigure b) shows that all of the distal apexes of the second sub-ring located at the distal end are not covered by the first membrane;
Fig. 9 is a schematic diagram showing the structure of the distal end of the main stent of the covered stent according to an embodiment of the present invention, where at least some areas of the second sub-ring located at the proximal end are non-fixedly connected to the first membrane;
Fig. 10 is a schematic diagram showing a partial structure of the main stent of the covered stent according to an embodiment of the present invention, where the subfigure a) shows that the back rib is connected to the second sub-ring, and the subfigure b) shows that the back rib is connected to both the second sub-ring and the first membrane;
Fig. 11a is a schematic diagram showing the structure of a covered stent according to an embodiment of the present invention, where coils are shown;
Fig. 11b is a schematic diagram showing the structure of a covered stent according to an embodiment of the present invention, where coils are arranged differently from that in Fig. 11a;
Fig. 12a is a schematic diagram showing the structure of a covered stent according to an embodiment of the present invention, where the restraining wire passes through the coils and is loosen;
Fig. 12b is a schematic diagram showing the structure of a covered stent according to an embodiment of the present invention, where the restraining wire is tense;
Fig. 13 is a schematic diagram showing the structure of the distal end of the first branch stent of the covered stent according to an embodiment of the present invention, where the subfigure a) shows that all of the distal apexes of the second ring located at the distal end are covered by the second membrane, the subfigure b) shows that all of the distal apexes of the second ring located at the distal end are not covered by the second membrane, and the subfigure c) shows that the second rings located at the distal end are not covered by the second membrane;
Fig. 14a is a schematic diagram showing the operation of the covered stent according to an embodiment of the present invention, where the restraining assembly applies a tensile force to the collapsed area, and the first branch stent has been implanted into the innominate artery;
Fig. 14b is a schematic diagram showing the operation of the covered stent according to an embodiment of the present invention, where the restraining assembly applies a tensile force to the collapsed area, the second branch stent has been implanted into the left common carotid artery, and the third branch stent has been implanted into the left subclavian artery;
Fig. 14c is a schematic diagram showing the operation of the covered stent according to an embodiment of the present invention, where the restraining assembly no longer applies a tensile force to the collapsed area, and the main stent has been completely released.

### List of reference signs:

100, main stent; 101, first interface; 102, second interface; 103, third interface; 110, collapsed area; 120, first ring; 121, first sub-ring; 122, second sub-ring; 130, first membrane; 140, back rib;
200, first branch stent; 210, second ring; 220, second membrane;
300, second branch stent;
400, third branch stent;
500, restraining assembly; 501, coil; 502, restraining wire;
610, first embedded support; 620, second embedded support;
11, aortic arch; 12, ascending aorta; 13, descending aorta; 14, innominate artery; 15, left common carotid artery; 16, left subclavian artery;
S1, anterior segment; S2, middle segment; S3, posterior segment.

### DETAILED DESCRIPTION

The embodiments of the present invention are described below through specific examples, and those skilled in the art can easily understand other advantages and effects of the present invention from the contents disclosed in this specification. The present invention can also be implemented or applied through other different embodiments, and various details in this specification can also be modified or changed based on different viewpoints and applications. It should be noted that the drawings provided in this embodiment are only to illustrate the basic concept of the present invention in a schematic way, so the drawings only show the components related to the present invention rather than the number, shape and the number of components in actual implementation. For dimension drawing, the type, quantity and proportion of each component can be changed at will in actual implementation, and the component layout may also be more complicated.

In addition, each embodiment of the following description has one or more technical features, but this does not mean that the person using the present invention must implement all the technical features in any embodiment at the same time, or can only implement some or all of the technical features of the different embodiments separately. In other words, under the premise of possible implementation, those skilled in the art can selectively implement some or all of the technical features in any embodiment or combinations thereof according to the disclosure of the present invention and depending on design specifications or implementation requirements, thereby increasing the flexibility of the implementation of the present invention.

As used in this specification, the singular forms "a", "an", and "the" include plural referents, and the plural forms "a plurality" include two or more referents unless the content clearly dictates otherwise. As used in this specification, the term "or" is generally employed in its sense including "and/or" unless the content clearly dictates otherwise, and the terms "installed", "connected", "coupled" shall be understood in a broad sense, for example, it may be a fixed connection, a detachable connection, or an integral connection. It can be a mechanical connection or an electrical connection. It can be directly connected, or indirectly connected through an intermediate medium, and it can be the internal communication between two elements or the interaction relationship between the two elements. For those of ordinary skill in the art, the specific meanings of the above terms in the present invention can be understood according to specific situations.

Herein, the terms "proximal" and "distal" refer to the positions and directions of the elements relative to each other as viewed from the heart after the covered stent is implanted into the human body, usually the proximal end refers to the end close to the heart, and the distal end refers to the end away from the heart.

To make the objectives, advantages and features of the present invention clearer, the present invention will be further described in detail below with reference to figures. It is noted that the figures are provided in a very simplified form not necessarily presented to scale, with their only intention to facilitate convenience and clarity in explaining the disclosed embodiments. Throughout the figures, like reference numerals indicate the same or analogous components or elements.

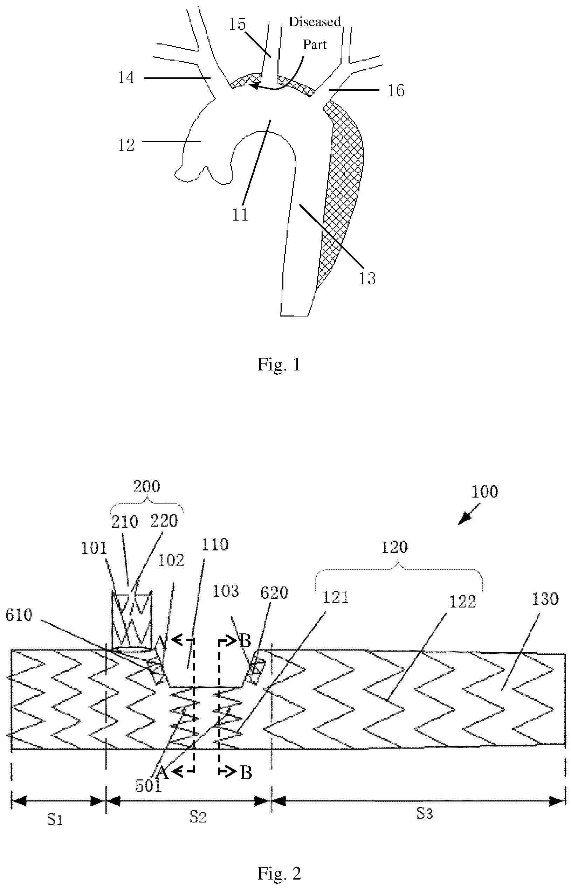

Fig. 1 is a schematic diagram showing a partial structure of human aorta, which mainly shows the aortic arch 11 which is curved as an arch in the upper part of the aorta. Taking the orientation shown in Fig. 1 as an example, the part of the aorta adjacent to the left side of the aortic arch 11 is the ascending aorta 12, and the part of the aorta adjacent to the right side of the aortic arch 11 is the descending aorta 13. The convex side of the aortic arch 11 has a greater curvature, and the concave side of the aorta arch 11 has a lesser curvature. There are three branch vessels on the greater curvature, along a direction from left to right, which are the innominate artery 14 (i.e., the brachiocephalic trunk artery), the left common carotid artery 15 and left subclavian artery 16. When diseases such as dissection or aneurysm occur at the aortic arch 11, a covered stent is required to be implanted in the aortic arch for treatment, but the covered stent should not block the three branch vessels.

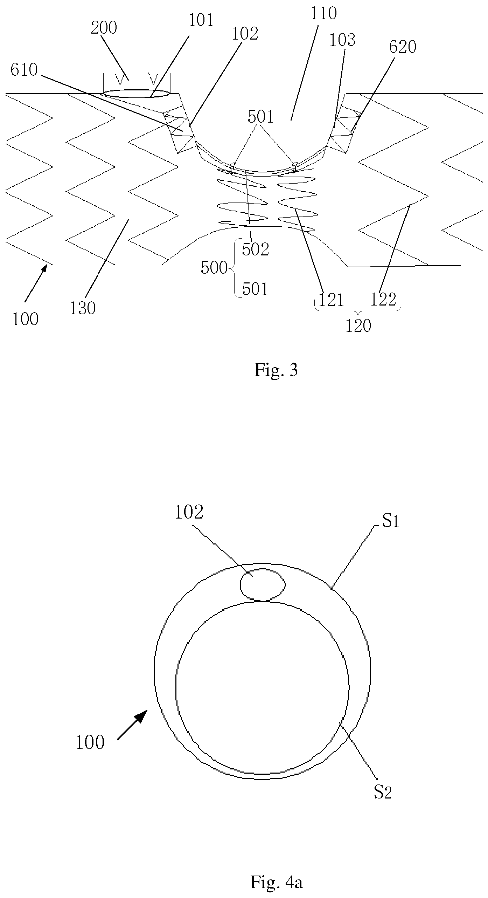

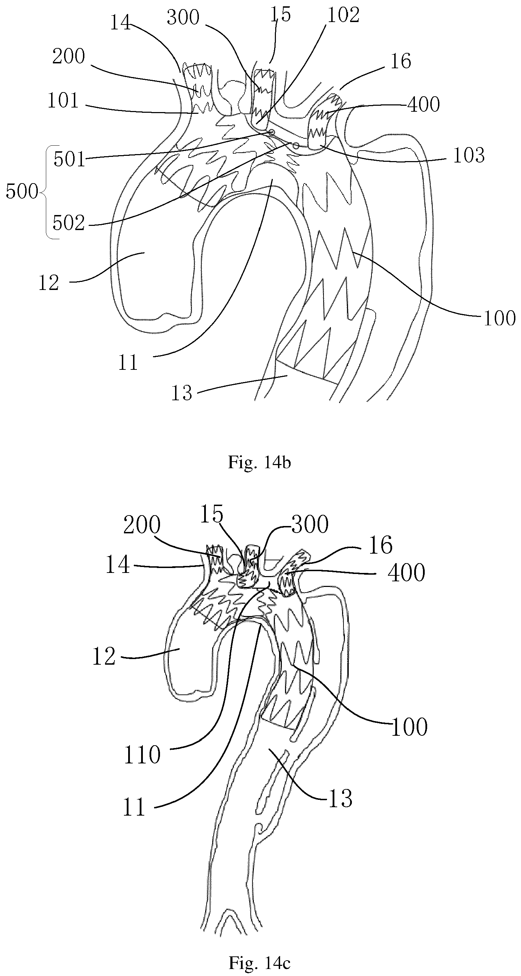

As shown in Figs. 2 and 3, an embodiment of the present invention provides a covered stent including a main stent 100 and a first branch stent 200. A first interface 101, a second interface 102 and a third interface 103 are arranged on a side surface of the main stent 100 and spaced along the axial direction of the main stent 100. The first branch stent 200 is provided at the first interface 101, and the first branch stent 200 is configured to be implanted into a branch blood vessel. For example, if a diseased part is at the aortic arch between the innominate artery 14 and the left common carotid artery 15 (as shown in Fig. 1), the first branch stent 200 is configured to be implanted into the innominate artery 14 (in this case, the first interface 101 is closest to the proximal end of the main stent 100). If a diseased part is between the left common carotid artery 15 and the left subclavian artery 16, the first branch stent 200 is configured to be implanted into the left subclavian artery 16 (in this case, the first interface 101 is closest to the distal end of the main stent 100). Here, the first branch stent 200 is implanted into the innominate artery 14 as an example for description. In this way, the second interface 102 is used to provide a blood flow channel for the left common carotid artery 15, and a second branch stent 300 can be provided at the second interface 102 (as shown in Figs. 14b and 14c). The third interface 103 is configured to provide a blood flow channel for the left subclavian artery, and a third branch stent 400 can be provided at the third interface 103 (as shown in Fig. 14c). That is to say, the side surface of the main stent 100 has two diametrically opposite sides, and the first interface 101, the second interface 102 and the third interface 103 are located on the same side. This side is referred to herein for convenience as the first side, and the opposite side is referred to as the second side. When the covered stent is implanted into the aorta, the first side faces with the greater curvature of the aortic arch 11, and the second side faces with the lesser curvature of the aorta arch 11. It can be understood that the side surface of the main stent 100 refers to the surface surrounding the axis of the main stent 100.

In practice, at least one of the second interface 102 and the third interface 103 is configured to receive a corresponding branch stent. Preferably, each of the second interface 102 and the third interface 103 is configured to receive a branch stent. In another word, the second interface 102 is provided with the second branch stent 300, and the third interface 103 is provided with the third branch stent 400. In this way, the covered stent further includes a restraining assembly 500 disposed on the main stent 100. The restraining assembly 500 is configured to apply to the main stent 100 a tensile force toward the inner cavity of the main stent 100, so that at least the portion of the main stent 100 where the second interface 102 and the third interface 103 are provided is recessed toward the inner cavity of the main stent 100. Generally, when the restraining assembly 500 provides the tensile force (i.e., when the restraining assembly 500 works), the second side of the main stent 100 corresponding to the second interface 102 and the third interface 103 is also is recessed. Therefore, the main stent 100 is in a recessed state when the restraining assembly 500 provides the tensile force, and the main stent 100 is in a natural state when the restraining assembly 500 does not provide the tensile force.

During operation, after the covered stent is implanted into the aorta and the first branch stent 200 is implanted into the innominate artery 14, if the restraining assembly 500 is kept working, the main stent 100 can be in the recessed state, and a gap is formed between the portion of the main stent 100 where the second interface 102 and the third interface 103 are provided and the blood vessel wall on the greater curvature of the aortic arch, so that the blood flow can flow from the second interface 102 and the third interface 103 into the gap and then into the branch blood vessel. In this case, the operator can sequentially implant the second branch stent 300 and the third branch stent 400. Since a gap is formed between the second interface 102 and the third interface 103 and the branch blood vessel, it is convenient for the guide wire to enter the corresponding branch blood vessel and establish a passageway for the access of the corresponding branch stent, and thus it is convenient for the implantation of the branch stents. In other words, in the embodiment of the present invention, the restraining assembly 500 is provided for temporarily reducing the diameter of the main stent 100 at the second interface 102 and the third interface 103, so that the gap formed between this area of the main stent 100 and the blood vessel wall on the greater curved side of the aortic arch 11 is large enough for conveniently establishing a passageway for the respective branch blood vessel, which improves the success rate for implantation of the branch stents, and also ensures the blood supply in the branch blood vessels during the implantation of the branch stents, avoiding branch vessel ischemia. Here, the first branch stent 200 is pre-connected to the main stent 100 and implanted in the innominate artery 14, which can enhance the anchorage stability of the covered stent so that the covered stent is prevented from displacement due to impact caused by blood flow.

It should be noted that, if only one of the second interface 102 and the third interface 103 is provided with a branch stent (for example, when the second branch stent 300 is provided at the second interface 102), the third interface 103 needs to be substantially aligned with the opening of the left subclavian artery 16. Otherwise, the third interface 103 may mismatch with the left subclavian artery 16 and cause the block of the left subclavian artery 16 when the restraining assembly 500 does not apply the tension force to the main stent 100 and the main stent 100 is fully released.

In view of this, a collapsed area 110 is preferably formed on the first side of the main stent 100, and the second interface 102 and the third interface 103 are both located in the collapsed area 110. The collapsed area 110 is configured to cover the opening of left common carotid artery 15 and the opening of left subclavian artery 16. In this way, even if only the second interface 102 is provided with a branch stent (i.e., the second branch stent 300), the ischemia of the left subclavian artery 16 can be avoided because the presence of the collapsed area 110 allows the blood flow to pass through the third interface 103 into the collapsed area 110 and then into the left subclavian artery 16 after the main stent 100 is completely released. The benefits of providing the collapsed area 110 are not limited to this. After the collapsed area 110 is defined, the restraining assembly 500 is preferably disposed at least partially at the collapsed area 110 and applies the tensile force to the collapsed area 110 so that the collapsed area 110 is further recessed toward the inner cavity of the main stent 100. As a result, it is possible to form a gap as large as required between the main stent 100 and the blood vessel wall on the greater curvature of the aortic arch in the case that the restraining assembly 500 provides a small tensile force. Moreover, the second interface 102 and/or the third interface 103 are prevented from being closed due to a large tensile force, which is more conducive to establishing a passageway for branch blood vessel. Further, the small tension force provided by the restraining assembly 500 can also reduce the degree of concavity of the area on the second side of the main stent 100 corresponding to the collapsed area 110 toward the inner cavity of the main stent 100, which is beneficial for the main stent 100 to adhere to the vessel wall on the lesser curvature of the aortic arch and avoiding the beak phenomenon when the restraining assembly 500 does not apply the tensile force and the main stent 100 is released completely. In addition, it should be noted that if the collapsed area 110 is provided on the main stent 100 while the restraining assembly 500 is cancelled, the collapsed area 110 will move toward the greater curvature of the aortic arch under the impact of blood flow, causing the collapsed area 110 to be too close to or adhere to the blood vessel wall on the greater curvature of the aortic arch, which is not conducive to the implantation of the branch stents, and even completely blocks the branch blood vessel.

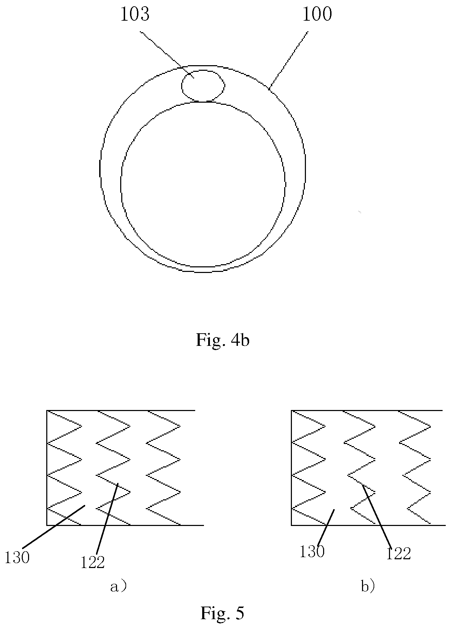

Preferably, the first interface 101 is arranged on a portion of the main stent 100 other than the collapsed area 110, and the second interface 102 and the third interface 103 are arranged at two opposite side surfaces of the collapsed area 110 (as shown in Figs. 2, 4a and 4b), so as to further prevent the second interface 102 and/or the third interface 103 from becoming closed under the action of the tensile force. When the diseased part occurs on the lesser curvature of the aortic arch, the first interface 101, the second interface 102 and the third interface 103 can all be arranged in the collapsed area 110.

Please continue to refer to Fig. 2, the main stent 100 is divided into three segments along the direction from the proximal end to the distal end, namely the anterior segment S1, the middle segment S2 and the posterior segment S3, respectively. The anterior segment S1 is used to be positioned in the ascending aorta 12, the middle segment S2 is used to be positioned in the aortic arch 11, and the posterior segment S3 is used to be positioned in the descending aorta 13. In this case, the first interface 101, the second interface 102 and the third interface 103 are all disposed on the middle segment S2. According to the biological anatomy of the aorta, the axial length of the anterior segment S1 is preferably 2~10 cm, and the axial length of the posterior segment S3 is preferably 1~20 cm. The outer diameter of the posterior segment S3 gradually decreases along the direction from the proximal end to the distal end of the main stent 100.

The specific configuration of the covered stent will be described as follows. It should be appreciated that the collapsed area 110 is formed on the main stent 100 as an example for the following description, but those skilled in the art can modify the following description to make it applicable to the case where the collapsed area 110 is not provided on the main stent 100.

Please continue to refer to Fig. 2, the main stent 100 includes a first ring 120 and a first membrane 130 at least partially covering the first ring 120. The first ring 120 includes a first sub-ring 121 and a second sub-ring 122. The number of the second sub-rings 122 is at least two. The at least two second sub-rings 122 are arranged at two axial sides of all the first sub-rings 121. It should be known that the number of the first sub-rings 121 is at least one. When the number of the first sub-rings 121 is more than one, a plurality of first sub-rings 121 are continuously arranged. In this embodiment, viewed from the axial direction of the main stent 100, the first sub-ring 121 may be a D-shaped ring having a straight edge facing the first side of the main stent 100, the second sub-ring 122 may be a circular ring or an oval-shaped ring. In this way, when the first membrane 130 covers the first sub-ring 121 and the second sub-ring 122, the collapsed area 110 is formed on the first side of the main stent 100.

In this embodiment, the first sub-ring 121 and the second sub-ring 122 are annular structures formed by a plurality of V-shaped rods connected end to end in sequence. Referring to Fig. 5, the axial lengths of the plurality of V-shaped rods located on a same second sub-ring 122 may be same or different. Preferably, the axial lengths of the plurality of V-shaped rods located on a same second sub-ring 122 are different, and preferably the lengths of the V-shaped rods is gradually increased along the direction from the second side to the first side, so that the rigidity of the main stent 100 is gradually increased in the direction from the second side to the first side. As a result, the second side is easier to bend and adapt to the lesser curvature of the aortic arch. In an alternative embodiment, the rigidity of the main stent 100 may be gradually increased in the direction from the second side to the first side in other ways. The first sub-ring 121 may be arranged in a similar manner to the second sub-ring.

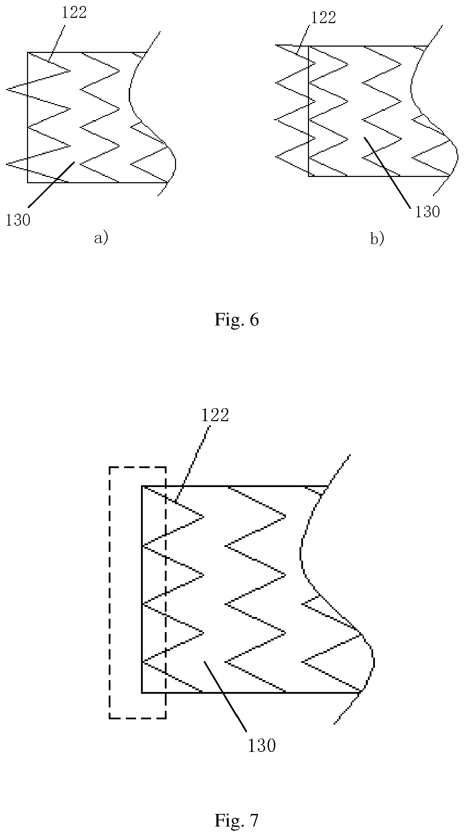

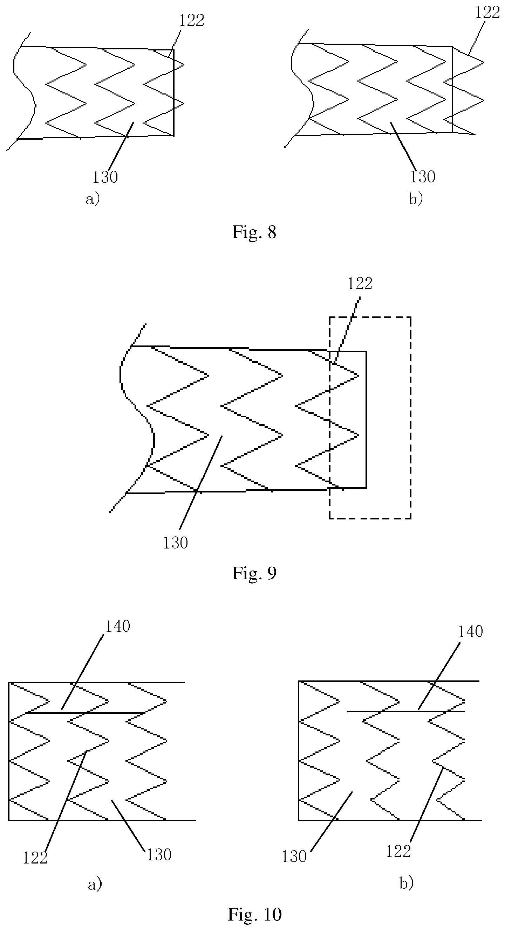

Optionally, at least a partial area of one of the second sub-rings 122 located at the proximal end of the main stent 100 is not covered by the first membrane 130. For example, please refer to the subfigure a) in Fig. 6, some of the proximal apexes of the second sub-ring 122 are covered by the first membrane 130, and the rest of the proximal apexes are exposed outside the first membrane 130. Alternatively, as shown in the subfigure b) in Fig. 6, all the proximal apexes of the second sub-ring 122 are exposed outside the first membrane 130. The advantage of this arrangement is that the proximal end of the main stent 100 has a bare segment which can be anchored in the ascending aorta 12, thereby reducing the possibility of displacement of the covered stent under the impact of blood flow. The proximal apex mentioned here refers to the apex of the second sub-ring 122 that is closer to the heart. Similarly, one of the second sub-rings 122 located at the distal end of the main stent 100 can also be configured similarly. As shown in Fig. 8, some of the distal apexes of the second sub-ring 122 are exposed outside the first membrane 130.

Please refer to Figs. 7 and 9 again, a part of the second sub-ring 122 located at the proximal end and/or the distal end of the main stent 100 is fixedly connected to the first membrane 130, and the other part of the area is not connected to the first membrane 130. Alternatively, the entire area of the second sub-ring 122 located at the proximal end and/or the distal end of the main stent 100 is not connected to the first membrane 130.

Further, please refer to Fig. 10, the main stent 100 further includes a back rib 140 extending along the axial direction of the main stent 100, and the back rib 140 is disposed on the first side of the main stent 100. The main stent 100 may include at least two back ribs 140 disposed at two sides of the collapsed area 110 so as to reduce the shortening rate of the main stent 100. The back ribs 140 can be disposed on the second sub-rings 122, and each of the back ribs 140 is connected to at least two of the second sub-rings 122. The back ribs 140 may also be connected to the first membrane 130.

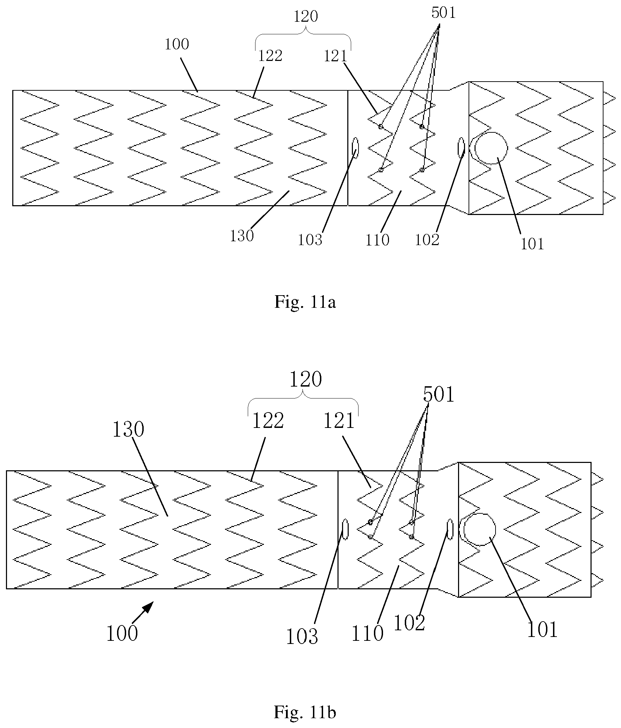

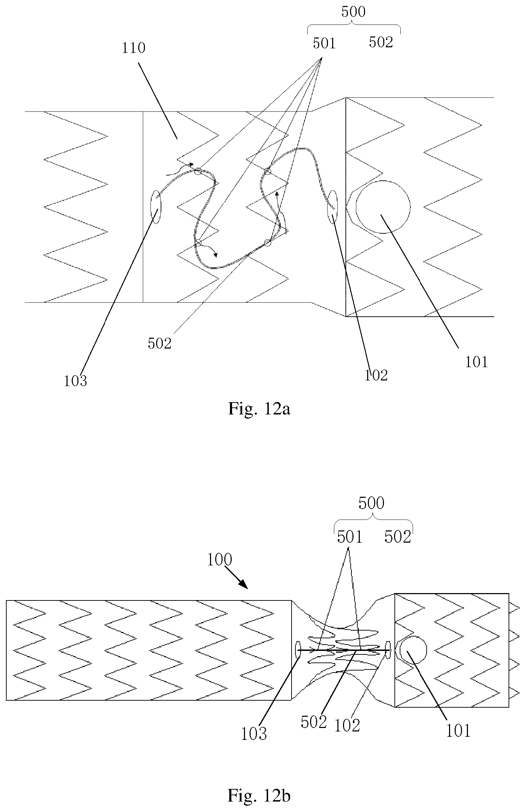

Please refer to Figs. 11a, 11b, 12a and 12b again, the restraining assembly 500 includes a plurality of coils 501 and a restraining wire 502, at least part of the coils 501 are located in the collapsed area 110 and connected to at least one of the first sub-ring 121. At least two of the coils 501 are provided on a same first sub-ring 121, and are sequentially arranged along the circumferential direction of the first sub-ring 121. The restraining wire 502 passes through all the coils 501 in sequence, for example, in the direction indicated by the arrow in Fig. 12a. When the restraining wire 502 is tense, the tensile force is applied from the restraining wire 502 through the coils 501 to the collapsed area 110 so that the main stent 100 is in the recessed state. When the restraining wire 502 is loosen, the restraining assembly 500 no longer applies the tensile force to the collapsed area 110, so that the main stent 100 is in the natural state. According to actual situations, the diameter of each of the coils 501 may be 1~4 mm, and each coil 501 is formed of a single-strand wire or a multi-strand wire.

It can be understood that the magnitude of the tensile force applied by the restraining assembly 500 to the collapsed area 110 depends on the number of the coils 501 disposed in the collapsed area 110 and the distance between two adjacent coils 501 on a same first sub-ring 111. For example, when the number of the coils 501 disposed in the collapsed area 110 remains unchanged, the tensile force applied by the restraining assembly 500 may be increased by enlarging the distance between two adjacent coils 501 located on a same first sub-ring 111. Specifically, please continue to refer to Figs. 11a and 11b, when two adjacent coils 501 on a same first sub-ring 111 are separated by a V-shaped structure (as shown in Fig. 11a), the maximum tensile force that the restraining assembly 500 can exert is F₁, and when two adjacent coils 501 on a same first sub-ring 111 are located on a same V-shaped structure (as shown in Fig. 11b), the maximum tensile force that the restraining assembly 500 can exert is F₂, where F₁ is greater than F₂.

In actual operation, the tensile force provided by the restraining assembly 500 should be within an appropriate range. If the tensile force is too small, the collapsed area 110 will move up under the impact of blood flow, resulting in a too small gap between the main stent 100 and the vessel wall on the greater curvature of the aortic arch. If the tensile force is too large, the restraining wire 502 is required to have a high strength. Moreover, the excessive tensile force will make the collapsed area 110 excessively recessed, and thus the inner diameter of the main stent 100 at the collapsed area 110 will be excessively reduced. As a result, the blood flow goes through the inner cavity of the main stent 100 may be reduced, causing the patient to suffer from lower extremity ischemia. It is not conducive to the subsequent release of the main stent 100, which is likely to cause the main stent 100 to be not in close contact with the blood vessel wall on the lesser curvature of the aortic arch, thereby resulting in the beak phenomenon.

Therefore, the restraining assembly 500 is configured to provide a tensile force that enables the inner diameter of the main stent 100 at the collapsed area 110 in the recessed state to be 20% to 80% of the inner diameter of the area in the natural state. In addition, in the natural state, the inner diameter d₂ of the main stent 100 at the collapsed area 110 is 50% to 90% of the maximum inner diameter of the main stent 100. For example, the inner diameter of the main stent 100 at the proximal end thereof is 44 mm, the inner diameter of the main stent 100 at the collapsed area 110 is 36 mm in the natural state, and the inner diameter (usually the minimum inner diameter) of the main stent 100 at the collapsed area 110 is about 20 mm in the recessed state.

As mentioned above, the second interface 102 and the third interface 103 are arranged on two opposite side surfaces of the collapsed area 110. In other words, the first interface 102 and the third interface 103 are both arranged between the first sub-rings 121 and the second sub-rings 122. Preferably, in this embodiment, a predetermined distance is defined between the second interface 102 and any one of the first rings 120 (i.e., any one of the first sub-rings 121 and the second sub-rings 122). The predetermined distance is not less than 2 mm, so that a space is formed for allowing the movements of the second interface 102. When the second branch stent 300 is provided, the allowed movements of the second interface 102 enables the second branch stent 300 to be implanted into the left common carotid artery 15 even if the second interface 102 is not aligned with the left common carotid artery. The third interface 103 can be configured similarly to the second interface 102. Specifically, a predetermined distance is defined between the third interface 103 and any one of the first rings 120 (i.e., any one of the first sub-rings 121 and the second sub-rings 122), and the predetermined distance is not less than 2 mm. In addition, the axial distance between the second interface 102 and the third interface 103 is 20~60 mm.

The covered stent further includes a first embedded support 610 and a second embedded support 620. The first embedded support 610 is disposed at the second interface 102, is located in the inner cavity of the main stent 100, and extends along the axial direction of the main stent 100. When the second branch stent 300 is disposed at the second interface 102, the second branch stent 300 can be detachably connected with the first embedded support 610 to reduce endoleak. Similarly, the second embedded support 620 is disposed at the third interface 103, is located in the inner cavity of the main stent 100, and extends along the axial direction of the main stent 100. When the third branch stent 400 is disposed at the third interface 103, the third branch stent 400 can be detachably connected with the second embedded support 620.

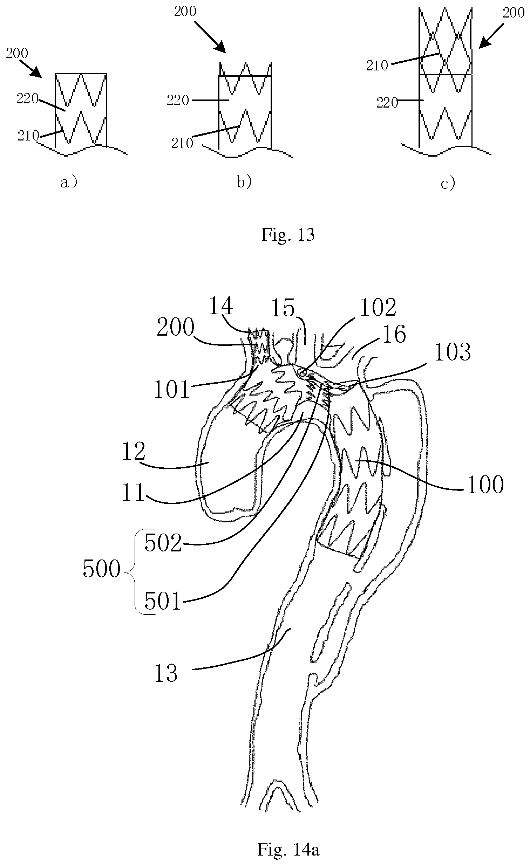

Referring back to Fig. 2 in conjunction with Fig. 13, the first branch stent 200 includes a second ring 210 and a second membrane at least partially covering the second ring 210. In an optional implementation, one of the second rings 210 located at the proximal end of the branch stent 200 is completely connected to the second membrane 220 in the circumferential direction, so that the first branch stent 200 is fixedly arranged at the first interface 101. In another optional implementation, one of the second rings 210 located at the proximal end of the branch stent 200 is partially connected with the second membrane 220 in the circumferential direction, so that the first branch stent 200 is movable at the first interface 101.

In some embodiments, as shown in the subfigure a) of Fig. 13, one of the second rings 210 located at the distal end of the first branch stent 200 may be completely covered by the second membrane 220. In other embodiments, as shown in the subfigure b) of Fig. 13, at least a partial area of one of the second rings 210 located at the distal end of the first branch stent 200 is not covered by the second membrane 220. In some embodiments, as shown in the subfigure c) in Fig. 13, at least two of the second rings 210 located at the distal end of the first branch stent 200 are not covered by the second membrane 220, so that the distal end of the first branch stent 200 can cross the bifurcation of the innominate artery 14 and enter the right vertebral artery (not annotated in the figure), without blocking the blood flow from passing through the bifurcation of the innominate artery 14, further enhancing the anchoring stability of the covered stent. Alternatively, at least a partial area of the second ring 210 located at the distal end of the first branch stent 200 is non-fixedly connected to the second membrane 220.

In addition, the covered stent further includes the second branch stent 300 and the third branch stent 400. The second branch stent 300 includes a third ring and a third membrane at least partially covering the third ring. The third branch stent 400 includes a fourth ring and a fourth membrane at least partially covering the fourth ring.

In addition, it should be noted that all the rings (including the first ring 120, the second ring 210, the third ring and the fourth ring) in the embodiments of the present invention can be made of metal materials. The rings can be made by cutting a tubular product or by braiding wires. All the membranes (including the first membrane 130, the second membrane 220, the third membrane and the fourth membrane) are made of polymer materials which are soft and compressible and have the property of isolating blood flow. The membrane can be placed on the corresponding ring by suture, heat fusion, or any other suitable manner. Since the first branch stent 200 and the main stent 100 are integrated together, the first membrane 130 and the second membrane 220 may be integrally formed, or the two may be formed separately and then connected in any suitable way such as by stitching or heat melting.

Referring to Figs. 14a to 14c, the operation of the covered stent will be described by taking a case where the diseased part occurs between the innominate artery 14 and the left common carotid artery 15 as an example. It should be noted that, during the delivery process, the covered stent is in a compressed state, and the restraining wire 502 of the restraining assembly 500 is tense. The procedure of the operation is described below.

First, referring to Fig. 14a, the covered stent is delivered to the predetermined portion of the aorta, and the pre-release of the main stent 100 and the release of the first branch stent 200 are performed, so that the anterior segment S1 of the main stent 100 is positioned in the ascending aorta 12, the middle segment S2 of the main stent 100 is positioned in the aortic arch 11, the posterior segment S3 of the main stent 100 is positioned in the descending aorta 13, and the first branch stent 200 is implanted in the innominate artery 14. At this time, the main stent 100 is in the recessed state, and a sufficiently large gap is formed between the collapsed area 110 and the blood vessel wall on the greater curvature of the aortic arch 11.

Then, referring to Fig. 14b, the main stent 100 remains in the recessed state, while the operator establishes a passageway for the second branch stent 300 at the second interface 102, and the second branch stent 300 is implanted into the left common carotid artery 15; and the operator also establishes a passageway for the third branch stent 400 at the third interface 103, and the third branch stent 400 is implanted into the left subclavian artery 16.

After that, the operator controls the restraining wire 502 to become loosen or pulls the restraining wire 502 to move it away from the coils 501. As a result, the restraining wire 502 no longer applies the tensile force to the main stent 100, so that the main stent 100 is completely released, and the second side of the main stent 100 is in close contact with the lesser curvature of the aortic arch 11 (shown in Fig. 14c).

Further, an embodiment of the present invention also provides a medical device, comprising a delivery mechanism and the covered stent as described above, the medical device is used to deliver and release the covered stent to a predetermined position, for example, the aortic arch.

Those skilled in the art should know that the two ends of the restraining wire penetrate into the inner cavity of the main stent through the second interface and the third interface respectively, and can be further extended outside of the delivery mechanism. Alternatively, at least one end of the restraining wire is connected to the controller of the delivery mechanism, so as to facilitate the operator's operation. It is known to those skilled in the art how to control the restraining wire in vitro, which will not be described in detail here.

Although the present invention is disclosed above, it is not limited thereto. It is apparent that those skilled in the art can make various modifications and variations to the present invention. Accordingly, the invention is intended to embrace all such modifications and variations if they fall within the scope of the appended claims.

## Claims

1. A covered stent, comprising a main stent (100) and a first branch stent (200), wherein a first interface (101), a second interface (102) and a third interface (103) are provided on a side surface of the main stent (100) and spaced along an axial direction of the main stent (100), the first interface (101) is configured to receive the first branch stent (200), the second interface (102) is configured to receive a second branch stent (300), and/or the third interface (103) is configured to receive a third branch stent (400); wherein the covered stent further comprises a restraining assembly (500) arranged on the main stent (100), and wherein the restraining assembly (500) is configured to apply to the main stent (100) a tensile force toward an inner cavity of the main stent (100), so that at least a portion of the main stent (100) that is provided with the second interface (102) and the third interface (103) is recessed toward the inner cavity of the main stent (100),
wherein a collapsed area (110) is formed at the side surface of the main stent (100), and the second interface (102) and the third interface (103) are both arranged in the collapsed area (110),
wherein the first interface (101) is located in a portion of the main stent (100) other than the collapsed area (110), and the second interface (102) and the third interface (103) are located on two opposite side surfaces of the collapsed area (110), and
wherein the restraining assembly (500) is disposed at least partially at the collapsed area (110), and is configured to apply the tensile force to the collapsed area (110) and to cause the collapsed area (110) to be further recessed toward the inner cavity of the main stent (100).

2. The covered stent according to claim 1, wherein the portion of the main stent (100) that is provided with the second interface (102) and the third interface (103) has an inner diameter for a recessed state that is 20% to 80% of an inner diameter for a natural state.

3. The covered stent according to claim 1, wherein the collapsed area (110) of the main stent (100) has an inner diameter for a natural state that is 50% to 90% of a maximum inner diameter of the main stent (100).

4. The covered stent according to claim 1, wherein the main stent (100) comprises a first ring (120) and a first membrane (130) at least partially covering the first ring (120), and wherein the first ring (120) comprises at least one first sub-ring (121) and at least two second sub-rings (122), the at least two second sub-rings (122) arranged on both axial sides of all of the at least one first sub-ring (121), and the at least one first sub-ring (121) and the at least two second sub-rings (122) form the collapsed area (110);
wherein the restraining assembly (500) comprises a plurality of coils (501) and a restraining wire (502), and wherein at least some of the coils (501) are disposed in the collapsed area (110) and connected to at least one of the at least one first sub-ring (121), and at least two of the coils (501) are arranged circumferentially on one of the at least one first sub-ring (121) along a circumferential direction of the main stent (100); the restraining wire (502) passes through all the coils (501); when the restraining wire (502) is tense, the tensile force is applied from the restraining wire (502) to the collapsed area (110) through the coils (501).

5. The covered stent according to claim 1, further comprising a second branch stent (300) and/or a third branch stent (400), and wherein the second branch stent (300) is configured to be detachably arranged at the second interface (102), and the third branch stent (400) is configured to be detachably arranged at the third interface (103).

6. The covered stent according to claim 4, wherein a predetermined distance is defined between the second interface (102) and any one of the at least one first sub-ring (121) and at least two second sub-rings (122); and/or
a predetermined distance is defined between the third interface (103) and any one of the at least one first sub-ring (121) and at least two second sub-rings (122).

7. The covered stent according to claim 1, further comprising a first embedded support (610) and/or a second embedded support (620), and wherein the first embedded support (610) is arranged at the first interface (101), is located in the inner cavity of the main stent (100), and is in communication with the inner cavity of the main stent (100); the second embedded support (620) is arranged at the third interface (103), is located in the inner cavity of the main stent (100), and is in communication with the inner cavity of the main stent (100).

8. A medical device, comprising a delivery mechanism and the covered stent according to any one of claims 1-7, wherein the delivery mechanism is configured to deliver and release the covered stent to a predetermined position.

## Patentansprüche

1. Ein gedeckter Stent, umfassend einen Hauptstent (100) und einen ersten Seitenaststent (200), wobei eine erste Schnittstelle (101), eine zweite Schnittstelle (102) und eine dritte Schnittstelle (103) an einer Seitenfläche des Hauptstents (100) vorgesehen und entlang einer axialen Richtung des Hauptstents (100) beabstandet sind, wobei die erste Schnittstelle (101) ausgebildet ist, den ersten Seitenaststent (200) aufzunehmen, die zweite Schnittstelle (102) ausgebildet ist, einen zweiten Seitenaststent (300) aufzunehmen, und/oder die dritte Schnittstelle (103) ausgebildet ist, einen dritten Seitenaststent (400) aufzunehmen; wobei der gedeckte Stent ferner eine Rückhalteanordnung (500) umfasst, die an dem Hauptstent (100) angeordnet ist, und wobei die Rückhalteanordnung (500) ausgebildet ist, eine Zugkraft auf den Hauptstent (100) in Richtung eines Innenhohlraums des Hauptstents (100) auszuüben, so dass zumindest ein Abschnitt des Hauptstents (100), der mit der zweiten Schnittstelle (102) und der dritten Schnittstelle (103) versehen ist, in Richtung des Innenhohlraums des Hauptstents (100) zurückgesetzt ist,
wobei ein kollabierter Bereich (110) an der Seitenfläche des Hauptstents (100) ausgebildet ist und die zweite Schnittstelle (102) und die dritte Schnittstelle (103) beide in dem kollabierten Bereich (110) angeordnet sind,
wobei die erste Schnittstelle (101) in einem Abschnitt des Hauptstents (100) angeordnet ist, der sich von dem kollabierten Bereich (110) unterscheidet, und die zweite Schnittstelle (102) und die dritte Schnittstelle (103) an zwei einander gegenüberliegenden Seitenflächen des kollabierten Bereichs (110) angeordnet sind, und
wobei die Rückhalteanordnung (500) zumindest teilweise an dem kollabierten Bereich (110) angeordnet ist und ausgebildet ist, die Zugkraft auf den kollabierten Bereich (110) auszuüben und zu bewirken, dass der kollabierte Bereich (110) weiter in Richtung des Innenhohlraums des Hauptstents (100) zurückgesetzt wird.

2. Der gedeckte Stent nach Anspruch 1, wobei der Abschnitt des Hauptstents (100), der mit der zweiten Schnittstelle (102) und der dritten Schnittstelle (103) versehen ist, einen Innendurchmesser für einen zurückgesetzten Zustand aufweist, der 20 % bis 80 % eines Innendurchmessers für einen natürlichen Zustand beträgt.

3. Der gedeckte Stent nach Anspruch 1, wobei der kollabierte Bereich (110) des Hauptstents (100) einen Innendurchmesser für einen natürlichen Zustand aufweist, der 50 % bis 90 % eines maximalen Innendurchmessers des Hauptstents (100) beträgt.

4. Der gedeckte Stent nach Anspruch 1, wobei der Hauptstent (100) einen ersten Ring (120) und eine erste Membran (130) umfasst, die den ersten Ring (120) zumindest teilweise bedeckt, und wobei der erste Ring (120) zumindest einen ersten Teilring (121) und zumindest zwei zweite Teilringe (122) umfasst, wobei die zumindest zwei zweiten Teilringe (122) auf beiden axialen Seiten des zumindest einen ersten Teilrings (121) angeordnet sind und der zumindest eine erste Teilring (121) und die zumindest zwei zweiten Teilringe (122) den kollabierten Bereich (110) bilden;
wobei die Rückhalteanordnung (500) eine Vielzahl von Ösen (501) und einen Rückhaltedraht (502) umfasst, und wobei zumindest einige der Ösen (501) in dem kollabierten Bereich (110) angeordnet und mit zumindest einem des zumindest einen ersten Teilrings (121) verbunden sind und zumindest zwei der Ösen (501) entlang einer Umfangsrichtung des Hauptstents (100) in Umfangsrichtung an einem des zumindest einen ersten Teilrings (121) angeordnet sind; der Rückhaltedraht (502) verläuft durch alle Ösen (501); und, wenn der Rückhaltedraht (502) gespannt ist, die Zugkraft von dem Rückhaltedraht (502) über die Ösen (501) auf den kollabierten Bereich (110) ausgeübt wird.

5. Der gedeckte Stent nach Anspruch 1, ferner umfassend einen zweiten Seitenaststent (300) und/oder einen dritten Seitenaststent (400), und wobei der zweite Seitenaststent (300) ausgebildet ist, lösbar an der zweiten Schnittstelle (102) angeordnet zu werden, und der dritte Seitenaststent (400) ausgebildet ist, lösbar an der dritten Schnittstelle (103) angeordnet zu werden.

6. Der gedeckte Stent nach Anspruch 4, wobei ein vorbestimmter Abstand zwischen der zweiten Schnittstelle (102) und einem beliebigen des zumindest einen ersten Teilrings (121) und der zumindest zwei zweiten Teilringe (122) definiert ist; und/oder
ein vorbestimmter Abstand zwischen der dritten Schnittstelle (103) und einem beliebigen des zumindest einen ersten Teilrings (121) und der zumindest zwei zweiten Teilringe (122) definiert ist.

7. Der gedeckte Stent nach Anspruch 1, ferner umfassend eine erste eingebettete Stütze (610) und/oder eine zweite eingebettete Stütze (620), und wobei die erste eingebettete Stütze (610) an der ersten Schnittstelle (101) angeordnet ist, in dem Innenhohlraum des Hauptstents (100) angeordnet ist und mit dem Innenhohlraum des Hauptstents (100) in Verbindung steht; die zweite eingebettete Stütze (620) an der dritten Schnittstelle (103) angeordnet ist, in dem Innenhohlraum des Hauptstents (100) angeordnet ist und mit dem Innenhohlraum des Hauptstents (100) in Verbindung steht.

8. Eine medizinische Vorrichtung, umfassend einen Zuführmechanismus und den gedeckten Stent nach einem der Ansprüche 1-7, wobei der Zuführmechanismus ausgebildet ist, den gedeckten Stent zu einer vorbestimmten Position zuzuführen und freizusetzen.

## Revendications

1. Stent couvert, comprenant un stent principal (100) et un premier stent de branche (200), dans lequel une première interface (101), une deuxième interface (102) et une troisième interface (103) sont prévues sur une surface latérale du stent principal (100) et espacées le long d'une direction axiale du stent principal (100), la première interface (101) étant configurée pour recevoir le premier stent de branche (200), la deuxième interface (102) étant configurée pour recevoir un deuxième stent de branche (300), et/ou la troisième interface (103) étant configurée pour recevoir un troisième stent de branche (400) ; dans lequel le stent couvert comprend en outre un ensemble de retenue (500) disposé sur le stent principal (100), et dans lequel l'ensemble de retenue (500) est configuré pour appliquer au stent principal (100) une force de traction vers une cavité interne du stent principal (100), de sorte qu'au moins une partie du stent principal (100) qui est pourvue de la deuxième interface (102) et de la troisième interface (103) soit en retrait vers la cavité interne du stent principal (100),
dans lequel une zone affaissée (110) est formée au niveau de la surface latérale du stent principal (100), et la deuxième interface (102) et la troisième interface (103) sont toutes deux disposées dans la zone affaissée (110),
dans lequel la première interface (101) est située dans une partie du stent principal (100) autre que la zone affaissée (110), et la deuxième interface (102) et la troisième interface (103) sont situées sur deux surfaces latérales opposées de la zone affaissée (110), et
dans lequel l'ensemble de retenue (500) est disposé au moins partiellement au niveau de la zone affaissée (110), et est configuré pour appliquer la force de traction à la zone affaissée (110) et pour amener la zone affaissée (110) à être davantage en retrait vers la cavité interne du stent principal (100).

2. Stent couvert selon la revendication 1, dans lequel la partie du stent principal (100) qui est pourvue de la deuxième interface (102) et de la troisième interface (103) présente un diamètre intérieur pour un état en retrait qui représente 20 % à 80 % d'un diamètre intérieur pour un état naturel.

3. Stent couvert selon la revendication 1, dans lequel la zone affaissée (110) du stent principal (100) présente un diamètre intérieur pour un état naturel qui représente 50 % à 90 % d'un diamètre intérieur maximal du stent principal (100).

4. Stent couvert selon la revendication 1, dans lequel le stent principal (100) comprend un premier anneau (120) et une première membrane (130) recouvrant au moins partiellement le premier anneau (120), et dans lequel le premier anneau (120) comprend au moins un premier sous-anneau (121) et au moins deux seconds sous-anneaux (122), les au moins deux seconds sous-anneaux (122) étant disposés de part et d'autre, dans la direction axiale, de l'ensemble de l'au moins un premier sous-anneau (121), et l'au moins un premier sous-anneau (121) et les au moins deux seconds sous-anneaux (122) formant la zone affaissée (110) ;
dans lequel l'ensemble de retenue (500) comprend une pluralité d'œillets (501) et un fil de retenue (502), et dans lequel au moins certains des œillets (501) sont disposés dans la zone affaissée (110) et reliés à au moins un premier sous-anneau (121), et au moins deux des œillets (501) sont disposés de manière circonférentielle sur l'un des au moins un premier sous-anneau (121) le long d'une direction circonférentielle du stent principal (100) ; le fil de retenue (502) passe à travers tous les œillets (501) ; et, lorsque le fil de retenue (502) est tendu, la force de traction est appliquée par le fil de retenue (502) à la zone affaissée (110) par l'intermédiaire des œillets (501).

5. Stent couvert selon la revendication 1, comprenant en outre un deuxième stent de branche (300) et/ou un troisième stent de branche (400), et dans lequel le deuxième stent de branche (300) est configuré pour être disposé de manière détachable au niveau de la deuxième interface (102), et le troisième stent de branche (400) est configuré pour être disposé de manière détachable au niveau de la troisième interface (103).

6. Stent couvert selon la revendication 4, dans lequel une distance prédéterminée est définie entre la deuxième interface (102) et l'un quelconque parmi l'au moins un premier sous-anneau (121) et les au moins deux seconds sous-anneaux (122) ; et/ou
une distance prédéterminée est définie entre la troisième interface (103) et l'un quelconque parmi l'au moins un premier sous-anneau (121) et les au moins deux seconds sous-anneaux (122).

7. Stent couvert selon la revendication 1, comprenant en outre un premier support encastré (610) et/ou un deuxième support encastré (620), et dans lequel le premier support encastré (610) est disposé au niveau de la première interface (101), est situé dans la cavité interne du stent principal (100), et est en communication avec la cavité interne du stent principal (100) ; et le deuxième support encastré (620) est disposé au niveau de la troisième interface (103), est situé dans la cavité interne du stent principal (100), et est en communication avec la cavité interne du stent principal (100).

8. Dispositif médical, comprenant un mécanisme d'acheminement et le stent couvert selon l'une quelconque des revendications 1 à 7, dans lequel le mécanisme d'acheminement est configuré pour acheminer et libérer le stent couvert au niveau d'une position prédéterminée.
